# EUROPEAN PATENT SPECIFICATION

(11) **EP 3 190 129 B1**
(45) Date of publication and mention of the grant of the patent: **08.01.2020**
(21) Application number: 15837573.3
(22) Date of filing: 02.09.2015
(51) Int. Cl.: C07K 19/00, A61K 38/26, A61P 25/24, A61P 25/28, A61P 43/00, C07K 14/605, A61K 38/28, A61K 38/22

(54) **CENTRALLY-ACTING PEPTIDE DERIVATIVE, AND PHARMACEUTICAL COMPOSITION**
ZENTRAL WIRKENDES PEPTIDDERIVAT UND PHARMAZEUTISCHE ZUSAMMENSETZUNG
DÉRIVÉ DE PEPTIDE À ACTION CENTRALE, ET COMPOSITION PHARMACEUTIQUE

(30) Priority: 02.09.2014 JP 2014177974; 10.09.2014 JP 2014184436
(43) Date of publication of application: 12.07.2017
(73) Proprietor: Tokyo University of Science Foundation, Tokyo 162-8601 (JP)
(72) Inventor: YAMASHITA, Chikamasa, Tokyo 162-8601 (JP); OKA, Jun-Ichiro, Tokyo 162-8601 (JP); HORIGUCHI, Michiko, Tokyo 162-8601 (JP); SASAKI-HAMADA, Sachie, Tokyo 162-8601 (JP)
(74) Representative: Isarpatent
(86) International application number: PCT/JP2015/074962
(87) International publication number: WO 2016/035820

(56) References cited:
- WO-A1-2010/063124
- WO-A2-2010/054326
- JP-A- 2009 545 543
- JP-A- 2010 043 001
- US-A1- 2009 030 178
- FONSECA S B ET AL: "Recent advances in the use of cell-penetrating peptides for medical and biological applications", ADVANCED DRUG DELIVERY REVIEWS, ELSEVIER, AMSTERDAM, NL, vol. 61, no. 11, 30 September 2009 (2009-09-30), pages 953-964, XP026666155, ISSN: 0169-409X, DOI: 10.1016/J.ADDR.2009.06.001 [retrieved on 2009-06-16]
- ALFREDO ERAZO-OLIVERAS ET AL: "Improving the Endosomal Escape of Cell-Penetrating Peptides and Their Cargos: Strategies and Challenges", PHARMACEUTICALS, vol. 5, no. 12, 1 November 2012 (2012-11-01), pages 1177-1209, XP55321157, DOI: 10.3390/ph5111177
- KATAYAMA, S. ET AL.: 'Acylation of octaarginine: Implication to the use of intracellular delivery vectors' JOURNAL OF CONTROLLED RELEASE vol. 149, 2011, pages 29 - 35, XP027593310
- TAKAYAMA, K. ET AL.: 'Effect of the Attachment of a Penetration Accelerating Sequence and the Influence of Hydrophobicity on Octaarginine- Mediated Intracellular Delivery' MOLECULAR PHARMACEUTICS vol. 9, 2012, pages 1222 - 1230, XP055365276
- TAKAYAMA, K. ET AL.: 'Enhanced intracellular delivery using arginine-rich peptides by the addition of penetration accelerating sequences (Pas' JOURNAL OF CONTROLLED RELEASE vol. 138, 2009, pages 128 - 133, XP026394864
- RYUJI NAKAMURA ET AL.: 'GLP-2 Keibi Toyo-yo Seizai no Chusu Sayo' DAI 130 KAI THE JAPANESE PHARMACOLOGICAL SOCIETY KANTO BUKAI PROGRAM YOSHISHU 03 July 2014, page 54, XP008185554
- BETTY R. LIU ET AL: "Endocytic Trafficking of Nanoparticles Delivered by Cell-penetrating Peptides Comprised of Nona-arginine and a Penetration Accelerating Sequence", PLOS ONE, vol. 8, no. 6, 26 June 2013 (2013-06-26), page e67100, XP55430950, DOI: 10.1371/journal.pone.0067100
- KAZUHIRO SHIGA ET AL: "Development of an intracellularly acting inhibitory peptide selective for PKN", BIOCHEMICAL JOURNAL, vol. 23, no. 2, 15 January 2010 (2010-01-15), pages 8596-543, XP55209914, ISSN: 0264-6021, DOI: 10.1042/BJ20090380
- LI-LI ZOU ET AL: "Cell-Penetrating Peptide-Mediated Therapeutic Molecule Delivery into the Central Nervous System", CURRENT NEUROPHARMACOLOGY, vol. 11, no. 2, 1 March 2013 (2013-03-01), pages 197-208, XP055506338, NL ISSN: 1570-159X, DOI: 10.2174/1570159X11311020006
- KASTIN A J ET AL: "INTERACTIONS OF GLUCAGON-LIKE PEPTIDE-1 (GLP-1) WITH THE BLOOD-BRAIN BARRIER", JOURNAL OF MOLECULAR NEUROSCIENCE, BIRKHAEUSER, CAMBRIDGE, MA, US, vol. 18, no. 1/02, 1 February 2002 (2002-02-01), pages 7-14, XP001085143, ISSN: 0895-8696, DOI: 10.1385/JMN:18:1-2:07
- TAKASHI IWAI ET AL: "Glucagon-like peptide-2 but not imipramine exhibits antidepressant-like effects in ACTH-treated mice", BEHAVIOURAL BRAIN RESEARCH, ELSEVIER, AMSTERDAM, NL, vol. 243, 9 January 2013 (2013-01-09), pages 153-157, XP028522956, ISSN: 0166-4328, DOI: 10.1016/J.BBR.2013.01.010 [retrieved on 2013-01-16]
- GEVAERT BERT ET AL: "Blood-brain barrier transport kinetics of the neuromedin peptides NMU, NMN, NMB and NT", NEUROPHARMACOLOGY, PERGAMON PRESS, OXFORD, GB, vol. 107, 1 April 2016 (2016-04-01), pages 460-470, XP029602111, ISSN: 0028-3908, DOI: 10.1016/J.NEUROPHARM.2016.03.051
- MIE KRISTENSEN ET AL: "Routes for Drug Translocation Across the Blood-Brain Barrier: Exploiting Peptides as Delivery Vectors", JOURNAL OF PHARMACEUTICAL SCIENCES, vol. 106, no. 9, 10 May 2017 (2017-05-10), pages 2326-2334, XP055507272, US ISSN: 0022-3549, DOI: 10.1016/j.xphs.2017.04.080
- SASAKI-HAMADA SACHIE ET AL: "Antidepressant-like effects exerted by the intranasal administration of a glucagon-like peptide-2 derivative containing cell-penetrating peptides and a penetration-accelerating sequence in mice", PEPTIDES, ELSEVIER, AMSTERDAM, NL, vol. 87, 25 November 2016 (2016-11-25), pages 64-70, XP029880491, ISSN: 0196-9781, DOI: 10.1016/J.PEPTIDES.2016.11.013
- Betty R. Liu ET AL: "Endocytic Trafficking of Nanoparticles Delivered by Cell-penetrating Peptides Comprised of Nona-arginine and a Penetration Accelerating Sequence", PLoS ONE, vol. 8, no. 6, 26 June 2013 (2013-06-26), page e67100, XP055430950, DOI: 10.1371/journal.pone.0067100

## Description

### Technical Field

The invention relates to a centrally-acting peptide derivative and a pharmaceutical composition.

### Background Art

In recent years, there has been a large amount of research on the pharmacological applications of the physiological activities of substances, such as peptides, that are derived from biological bodies. For example, it is known that the glucagon-like peptide-1 (GLP-1), which is a peptide that is derived from proglucagon and formed from 37 amino acid residues, and the glucagon-like peptide-2 (GLP-2), which is a peptide that is derived from proglucagon and formed from 33 amino acid residues, bind to a G-protein-coupled receptor (GPCR) and activate signaling transduction.

Regarding the pharmacological activity of GLP-1 (amide activity types 7-37 and 7-36 are present) in the brain, there have been reports of an effect of alleviating learning disorders (for example, Non-Patent Documents 1 and 2).

Regarding the pharmacological activity of GLP-2 in the brain, there have been reports of effects of anti-depresssive activity even in treatment resistant depression model animals, lowering blood pressure and alleviating learning disorders (for example, Non-Patent Documents 3 to 9).

Further, it has been reported that neuromedin U (NmU), which is a peptide formed of 23 amino acid residues, binds to a GPCR in the brain and exerts an effect of alleviating learning disorders (for example, Non-Patent Document 10).

Peptides having a central activity such as opioid peptides, oxytocin, leptin, neuropeptide Y, orexin and insulin, and antibody drugs, have also been developed. Patent Document 1 relates to a GLP-1 peptide fused to a cell-penetrating peptide. Non-Patent Document 11 relates to a cell penetrating peptide that can be improved to escape from endosomal entrapment. Non-Patent Document 12 reviews cell-penetrating peptides. Non-Patent Document 13 relates to PasR8 conjugates with three bioactive species. Non-Patent Document 14 relates to nanoparticles which also act on the CNS. Non-Patent Document 15 relates to PKN inhibitors. Patent Document 2 relates to peptides targeting various peptide hormones to the CNS.

### [Prior Art Document]

Non-Patent Document 1: Neuroscience Research 64(2009)67-74
Non-Patent Document 2: Journal of Neuroscience Research 92(2014)446-454
Non-Patent Document 3: Behavioural Brain Research 204(2009)235-240
Non-Patent Document 4: Neuroscience 212(2012)140-148
Non-Patent Document 5: Life Sciences 93(2013)889-896
Non-Patent Document 6: Neuroscience Letters 550(2013)104-108
Non-Patent Document 7: Behavioural Brain Research 243(2013)153-157
Non-Patent Document 8: Neuropeptides 49(2015)7-14
Non-Patent Document 9: Neuroscience 294(2015)156-165
Non-Patent Document 10: Neuroscience Research 61(2008)113-119
Non-Patent Document 11: Advanced Drug Delivery Reviews vol. 61, no. 11 (2009) 60-61
Non-Patent Document 12: Pharmaceuticals vol. 5, no. 12 (2012) 1177 - 1209
Non-Patent Document 13: Molecular Pharmaceutics vol. 9 (2012) 1222 - 1230
Non-Patent Document 14: Plos One vol. 8, no. 6 (2013) e67100
Non-Patent Document 15: Biochemical Journal vol. 23, no. 2 (2010), 8596-543

Patent Document 1: WO 2010/054326 A1
Patent Document 2: WO 2010/063124 A1

### [Summary]

### [Problem to be Solved by the Disclosure]

It is anticipated that substances derived from a biological body, such as peptides, may be used as an active component of a drug with fewer side effects than previous drugs. It is desirable for drugs that need to be taken on a daily basis to be administered in a less invasive manner. Therefore, development of a nasal formulation as a dosage form that is adaptable for less-invasive administration has been studied. However, there have been problems in that a peptide that has been administered in an intranasal manner does not easily pass the blood-brain barrier, or that the effect of a peptide is lost within a short period of time due to enzymatic decomposition in a biological body. The inventors have focused on intranasal administration, which is a promising administration in terms of being less invasive and capable of delivering a peptide to the central nervous system, and have enabled the delivery of a centrally-acting peptide to the brain in an efficient manner by adding a peptide having a specific sequence to the centrally-acting peptide. Specifically, the invention aims to provide a centrally-acting peptide derivative having a superior ability to migrate to the central nervous system, and a pharmaceutical composition including the centrally-acting peptide derivative. The invention is defined by the appended claims.

### Disclosed is also:

penetrating sequence portion, and an endosomal-escape portion.
<2> The centrally-acting peptide derivative according to <1>, wherein the centrally-acting portion is an amino acid sequence portion that is derived from a peptide.
<3> The centrally-acting peptide derivative according to <2>, wherein the amino acid sequence portion is derived from a peptide that is GLP-1, GLP-2, neuromedin U, an opioid peptide, oxytocin, leptin, orexin, neuropeptide Y or insulin.
<4> The centrally-acting peptide derivative according to <2> or <3>, wherein the amino acid sequence portion is derived from a peptide selected from the following (a1), (a2), (a3) or (b):
   (a1) a peptide comprising an amino acid sequence represented by His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp (sequence number 1);
   (a2) a peptide comprising an amino acid sequences represented by His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-NH₂ (sequence number 2);
   (a3) a peptide comprising an amino acid sequence represented by Tyr-Lys-Val-Asn-Glu-Tyr-Gln-Gly-Pro-Val-Ala-Pro-Ser-Gly-Gly-Phe-Phe-Leu-Phe-Arg-Pro-Arg-Asn-NH₂ (neuromedin U, sequence number 3); and
   (b) a peptide comprising an amino acid sequence represented by any of (a1) to (a3) in which one or more amino acid residues are deleted, replaced or added, the peptide having a centrally-acting activity.
<5> The centrally-acting peptide derivative according to any one of <1> to <4>, used for treatment of a neuropsychiatric disorder.
<6> The centrally-acting peptide derivative according to any one of < 1> to <4>, used for treatment of depression or a learning disorder.
<7> A pharmaceutical composition, comprising the centrally-acting peptide derivative according to any one of claim 1 to <6> as an active ingredient.
<8> The pharmaceutical composition according to <7>, used for treatment of a neuropsychiatric disorder.
<9> The pharmaceutical composition according to <7> or <8>, used for treatment of depression or a learning disorder.
<10> A nasal formulation comprising the centrally-acting peptide derivative according to any one of <1> to < 6> as an active ingredient.
<11> The nasal formulation according to <10>, used for treatment of a neuropsychiatric disorder.
<12> The nasal formulation according to <10> or <11>, used for treatment of depression or a learning disorder.
<13> Use of a pharmaceutical composition for intranasal administration, the pharmaceutical composition comprising the centrally-acting peptide derivative according to any one of <1> to <6> as an active ingredient.
<14> A method of stabilizing a centrally-acting peptide, the method comprising adding a cell-penetrating sequence portion and an endosomal-escape portion to a centrally-acting peptide.

### [Effect of the Invention]

According to the invention, it is possible to provide a centrally-acting peptide derivative having a superior ability to migrate to the central nervous system, and a pharmaceutical composition including the centrally-acting peptide derivative.

### [Brief Explanation of the Drawings]

Fig. 1 shows the result of a forced-swim test on a rat administered with a GLP-2 derivative.
Fig. 2 shows the result of a forced-swim test on a rat administered with GLP-2 alone.

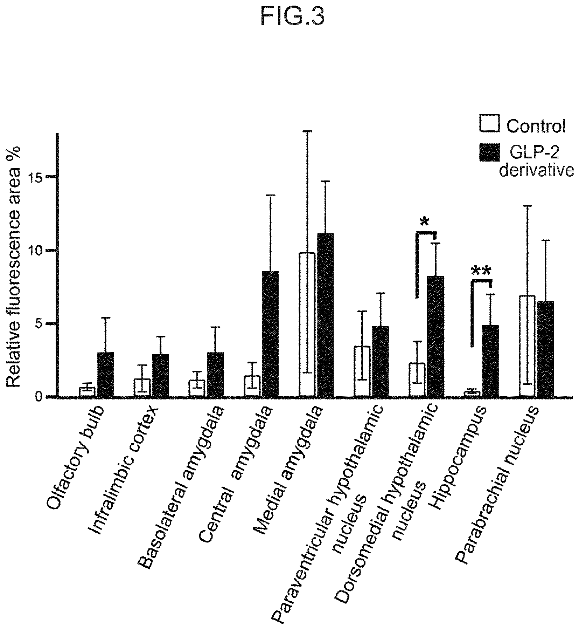

Fig. 3 shows the distribution of a GLP-2 derivative in the brain of a rat.
Fig. 4 shows the result of a forced-swim test on a mouse administered with a GLP-2 derivative.
Fig. 5 shows the result of a tail suspension test on a mouse administered with a GLP-2 derivative.
Fig. 6 shows the result of a forced-swim test on a treatment-resistant depression model mouse administered with a GLP-2 derivative.
Fig. 7 shows the result of a Y-maze test on a learning disorder model mouse administered with a GLP-1 derivative.
Fig. 8 shows the result of a Y-maze test on a learning disorder model mouse administered with an NmU derivative.
Fig. 9 shows the result of a stability test of DPP-4, which is an enzyme that decomposes a GLP-2 derivative.

### [Embodiments for Implementing the Invention]

In the following, embodiments of the invention are explained. The explanation and the embodiments are exemplify the invention, and do not limit the scope of the invention.

Numerical ranges indicated as "from A to B" described herein include A and B as a minimum value and a maximum value, respectively.

The left side of the amino acid sequence refers to the N-terminal, and the amino acid residue may be indicated by a one-letter abbreviation (for example, G for glycine residue) or a three-letter abbreviation (for example, Gly for glycine residue).

"Treatment" as described herein refers not only to an activity or an effect that quenches or alleviates a symptom, but also to an activity or an effect that suppresses aggravation of the symptom.

An "anti-depressive activity" or "anti-depressive effect" refers not only to an activity or an effect that quenches a symptom of depression, but also to an activity or an effect that suppresses aggravation of the symptom.

A "learning disorder-alleviating activity" or "learning disorder-alleviating effect" refers not only to an activity or an effect that quenches a symptom of a learning disorder, but also to an activity or an effect that suppresses aggravation of the symptom.

### <Centrally-acting peptide derivative>

The centrally-acting peptide derivative of the invention has a centrally-active portion, a cell-penetrating sequence portion and an endosomal-escape portion. The inventors have studied on a method to deliver a centrally-acting peptide to the brain in an expeditious manner before the peptide decomposes in a body, and to deliver a centrally-acting peptide to the brain in an efficient manner. As a result, the inventors have found that the ability of a peptide to migrate to a brain is significantly improved when the peptide is intranasally administered as derivative having a centrally-active portion, a cell-penetrating sequence portion and an endosomal-escape portion, as compared with the case in which the centrally-active portion alone is intranasally administered.

The intended purpose of the centrally-acting peptide derivative is not limited, as long as it employs a pharmacological activity that is expressed by the centrally-acting portion upon acting on the central nervous system. Examples of the pharmacological activity include an anti-depression activity, a learning disorder-alleviating activity, an anti-anxiety activity, a feeding suppression activity, a cognitive disorder-alleviating activity, a blood-pressure-lowering activity, an analgesic activity, a sleep-inducing activity, and an anti-epileptic activity. Therefore, the centrally-acting peptide derivative is suitably used as a drug for the treatment of neuropsychiatric disorders, such as an anti-depression agent, a learning disorder-alleviating agent, an anti-anxiety agent, a feeding suppression agent, a cognitive disorder-alleviating agent, a blood-pressure-lowering agent, an analgesic agent, a sleep-inducing agent, and an anti-epileptic agent.

### (Centrally-acting portion)

The centrally-acting portion in the centrally-acting peptide derivative is not particularly limited, as long as it exerts a pharmacological activity by acting on the central nervous system. Addition of a cell-penetrating sequence portion and an endosomal-escape portion to a centrally-acting portion may be performed by a known process without particular restriction.

In an embodiment of the centrally-acting peptide derivative, the centrally-acting portion is an amino acid sequence portion derived from a centrally-acting peptide (also referred to as a centrally-acting peptide portion). Examples of the centrally-acting peptide include GLP-1, GLP-2, neuromedin U, opioid peptide (such as enkephalin and dynorphin), oxytocin, leptin, orexin, neuropeptide Y and insulin.

In an embodiment of the centrally-acting peptide derivative, the centrally-acting portion is a peptide portion derived from a peptide that is any of the following (a1) to (a3) or (b).
(a1) a peptide comprising an amino acid sequence represented by HADGSFSDEMNTILDNLAARDFINWLIQTKITD (GLP-2, sequence number 1)
(a2) a peptide comprising an amino acid sequences represented by HAEGTFTSDVSSYLEGQAAKEFIAWLVKGR-NH₂ (GLP-1, 7-36 amide, sequence number 2)
(a3) a peptide comprising an amino acid sequence represented by YKVNEYQGPVAPSGGFFLFRPRN-NH₂ (neuromedin U, sequence number 3)
(b) a peptide comprising an amino acid sequence represented by any of (a1) to (a3) in which one or more amino acid residues are deleted, replaced or added, the peptide having a centrally-acting activity.

Among these peptides, GLP-2 exerts an anti-depression activity and a blood-pressure-lowering activity, GLP-1 exerts a learning disorder-alleviating activity, and neuromedin U exerts a learning disorder-alleviating activity. Therefore, these peptides are useful as a centrally-acting portion of the centrally-acting peptide derivative.

When a peptide is formed of an amino acid sequence of a specific peptide and an amino acid sequence of a different peptide, the term "amino acid sequence derived from the specific peptide" refers to a portion of the peptide corresponding to the amino acid sequence of the specific peptide.

When the centrally-acting portion is derived from "(b) a peptide including an amino acid sequence represented by any of (a1) to (a3) in which one or more amino acid residues are deleted, replaced or added", the number of the amino acid residues to be deleted, replaced or added is not particularly limited as long as the centrally-acting peptide portion exert the effect of the invention. For example, the number of the amino acid residues to be deleted, replaced or added may be from 1 to 10, preferably from 1 to 5, more preferably from 1 to 3. From the viewpoint of synthetic efficiency, handleability, stability and the like, the total number of the amino acid residues of the peptide (b) is preferably 40 or less, more preferably 35 or less, further preferably 33 or less.

### (Cell-penetrating sequence portion)

The centrally-acting peptide derivative has a cell-penetrating sequence portion. The cell-penetrating sequence portion is formed of an amino acid sequence of a cell-penetrating peptide (CPP). The cell-penetrating peptide refers to a peptide having an activity to penetrate a cell membrane. The cell-penetrating sequence portion introduces the centrally-acting peptide derivative into a cell by its cell-penetrating activity. It is considered that the centrally-acting peptide derivative is delivered to the brain via nasal membrane, olfactory epithelium or the like, mainly through the axonal transport or the like, and is distributed therein.

The structure or the mechanism of penetration of a cell-penetrating peptide that constitutes the cell-penetrating sequence portion is not particularly limited, as long as it has an activity to introduce the centrally-acting peptide derivative into a cell.

Examples of the cell-penetrating peptide include oligoarginine (Rn, n is the number of arginine residues ranging from 6 to 12), Angiopep-5(RFFYGGSRGKRNNFRTEEY, sequence number 4), Antp (RQIKIWFQNRRMKWKK, sequence number 5), Bac (YGRKKRRQRRR, sequence number 6), BR1 (RAGLQFPVGRLLR, sequence number 7), BR2 (RAGLQFPVGRLLRRLLR, sequence number 8), Buf IIb[BR3] (RAGLQFPVGRLLRRLLRRLLR, sequence number 9), SH-CPPP-2 (KLPVM, sequence number 10), CyLoP-1(CRWRWKCCKK, sequence number 11), Cys-Antp (CRQIKIWFQNRRMKWKK, sequence number 12), Cys-pVEC (CLLIILRRRIRKQAHAHKS, sequence number 13), Cys-SAP (CVRLPPPVRLPPPVRLPPP, sequence number 14), Cys-SAPr (CPPPLRVPPPLRVPPPLRV, sequence number 15), Cys-TAT (CYGRKKRRQRRR, sequence number 16), Cys-TP10 (CAGYLLGKINLKALAALAKKIL, sequence number 17), Cys-TP10K (CAGYLLGKINKLKALAALAKKIL, sequence number 18), FAK (FAKLAARLYRKALARQLGVAA, sequence number 19), H9(HHHHHHHHH, sequence number 20), HC-[poly(K)] (QYIKANSKFIGITEL-poly(K), sequence number 21), hLF (KCFQWQRNMRKVRGPPVSCIKR, sequence number 22), HR9 (CHHHHHHRRRRRRRRRHHHHHC, sequence number 23), IR9 (GLFEAIEGFIENGWEGMIDGWYGRRRRRRRRR, sequence number 24), JNKI-1D-TAT (TDQSRPVQPFLNLTTPRKPRRPPRRRQRRKKRG, sequence number 25), K9(KKKKKKKKK, sequence number 26), low molecular weight protamine (LMWP) (VSRRRRRRGGRRRR, sequence number 27), MAP(KLALKLALKALKAALKLA, sequence number 28), Mitoparan (INLKKLAKL(Aib)KKIL, sequence number 29), m-N2-10-1(MTS-C) (HYSRLEICNL-AAVALLPAVLLALLP, sequence number 30), m-N2-10-1(MTS-N) (AAVALLPAVLLALLP-HYSRLEICNL, sequence number 31), m-N2-10-1 (NIV-C) (HYSRLEICNL-GRKKRRQRRRPPQ, sequence number 32), m-N2-10-1 (NIV-N) (GRKKRRQRRRPPQ-HYSRLEICNL, sequence number 33), m-N2-10-1 (OR-C) (RRRRRRRR-HYSRLEICNL, sequence number 34), m-N2-10-1 (OR-N) (HYSRLEICNL-RRRRRRRR, sequence number 35), MPG-8 (AFLGWLGAWGTMGWSPKKKRK-Cysteamide, sequence number 36), MPGα (GALFLAAALSLMGLWSQPKKKRKV-NH-CH₂-CH₂-SH, sequence number 37), MPGβ (GALFLGAALSLMGLWSQPKKKRKV-NH-CH₂-CH₂-SH, sequence number 38), NrTP1 (YKQCHKKGGKKGSG, sequence number 39), NrTP2 (YKQCHKKGG-Ahx-KKGSG, sequence number 40), NrTP5 (YKGCHKKGGKKGSG, sequence number 41), NrTP6 (YKQSHKKGGKKGSG, sequence number 42), NrTP7 (YRQSHRRGGRRGSG, sequence number 43), MPG HIV-gp41/SV40 T-antigen (GALFLGFLGAAGSTMGAWSQPKKKRKV, sequence number 44), NrTP8(WKQSHKKGGKKGSG, sequence number 45), PAMAM-PEG-Angiopep-2 (PFFYGGSGGNRNNYLREEY, sequence number 46), PEI-TAT (TGRKKRRQRRR-PEI, sequence number 47), penetratin (43-58) (RQIKIWFQNRRMKWKK, sequence number 48), penetratin (RQIKIWFQNRRMKWKKK, sequence number 49), Pep-1 HIV-reverse transcriptase/SV40 T-antigen (KETWWETWWTEWSQPKKKRKV, sequence number 50), Pep1 (KETWWETWWTEWSQPKKKRKV, sequence number 51), pepM residues 45-72 of DENV-2 C protein (KLFMALVAFLRFLTIPPTAGILKRWGTI, sequence number 52), pepR residues 67-100 of DENV-2 C protein (LKRWGTIKKSKAINVLRGFRKEIGRMLNILNRRRR, sequence number 53), peptide derived from protamine (RRRRRRGGRRRRG, sequence number 54), peptide vascular endothelial cadherin (pVEC) (LLIILRRRIRKQAHAHSK, sequence number 55), PF14 (AGYLLGKLLOOLAAAALOOLL, O represents any amino acid residue, sequence number 56), PR9 (FFLIPKGRRRRRRRRR, sequence number 57), protamine (VSRRRRRRGGRRRRK, sequence number 58), PYC36L-TAT (GRKKRRQRRRGGLQGRRRQGYQSIKP, sequence number 59), R6W3 based on penetratin (RRWWRRWRR, sequence number 60), R9-hLF (RRRRRRRRRKCFQWQRNMRKVRGPPVSCIKR, sequence number 61), RA9 (RRAARRARR, sequence number 62), RH9(RRHHRRHRR, sequence number 63), RL9(RRLLRRLRR, sequence number 64), RVG-9R (YTIWMPENPRPGTPCDIFTNSRGKRASNG-9R, sequence number 65), RW9 (RRWWRRWRR, sequence number 66), S8 (SSSSSSSSK, sequence number 67), SAP (VRLPPPVRLPPPVRLPPP, sequence number 68), SAPr (PPPLRVPPPLRVPPPLRV, sequence number 69), SynB1 (RGGRLSYSRRRFSTSTGR, sequence number 70), SynB3 (RRLSYSRRRF, sequence number 71), Xentry (LCLRPVG, sequence number 72), YARA (YARAAARQARAKALARQLGVAA, sequence number 73), TAT (47-57) (GRKKRRQRRRP, sequence number 74), TAT (48-60) (GRKKRRQRRRPPQ, sequence number 75), TAT (49-57) (RKKRRQRRR, sequence number 76), TAT-D (GRRRQRRKKRG, sequence number 77), TAT-L (GRKKRRQRRRG, sequence number 78), TAT-PEG-Cholesterol (YGRKKRRQRRR-PEG-cholesterol, sequence number 79), TP-10 (AGYLLGKINLKALAALAKKIL, sequence number 80), TP-10K (AGYLLGKINKLKALAALAKKIL, sequence number 81), transportan (GWTLNSAGYLLGKINLKALAALAKKIL, sequence number 82), CAD-2 (GLWRALWRLLRSLWRLLWKA-NH-CH₂-CH²-SH, sequence number 83), PF6 (AGYLLGK(K(K2(TFQ4)))INLKALAALAKKIL, sequence number 84), and polyarginine derivative (RRRQRRKRRRQ, sequence number 85).

Among these cell-penetrating peptides, from the viewpoint of affinity with a cell membrane and ease of synthesis, a cell-penetrating peptide formed of an amino acid sequence that is rich in basic amino acid residues such as arginine, lycine, histidine and triptophan (for example, half or more of the total amino acid residues are basic amino acid residues) is preferred. Examples of such cell-penetrating peptides include oligoarginine (Rn, n is the number of arginine residues ranging from 6 to 12) and TAT derived from a Tat protein of human immunodeficiency virus 1 (HIV-1). A cell-penetrating peptide formed of an amino acid sequence that is rich in basic amino acid residues is considered to induce macropinocytosis, a form of endocytosis that is a process of a cell to take in extracellular substance. As a result, it is considered that the centrally-acting peptide derivative is introduced into a cell in a more efficient manner.

From the viewpoint of the efficiency of cell penetration of the centrally-acting peptide derivative, the cell-penetrating peptide is preferably a peptide in which half or more of the total amino acid residues are arginine residues, more preferably oligoarginine formed of 6 to 12 arginine residues, further preferably oligoarginine formed of 7 to 9 arginine residues, yet further preferably oligoarginine formed of 8 arginine residues.

### (Endosomal-escape portion)

The centrally-acting peptide derivative has an endosomal-escape portion. An endosomal-escape portion is considered to shorten the time for the centrally-acting peptide derivative to remain in a cell, and enables escape from the endosome in a shorter period. As a result, it is considered to become possible to expedite the delivery of the centrally-acting peptide derivative to the brain and the distribution of the centrally-acting peptide derivative in the brain.

The structure or the mechanism of endosomal escape of the endosomal-escape portion is not specifically limited, as long as it has an activity of promoting the escape of the centrally-acting peptide derivative from an endosome. Examples of the endosomal-escape portion include an acetyl group (CₙH₂ₙ₊₁Co-, n=3∼9) and a penetration accelerating sequence (PAS) such as FFLIPKG (sequence number 86), LILIG (sequence number 87), FFG (sequence number 88), FFFFG (sequence number 89) and FFFFFFG (sequence number 90). From the viewpoint of the function of causing endosomal escape, a PAS is a preferred endosomal escape portion.

The position of the cell-penetrating sequence portion and the endosomal-escape portion in the centrally-acting peptide derivative is not particularly restricted. For example, the cell-penetrating sequence portion may be positioned closer to the centrally-acting portion than the endosomal-escape portion, or the endosomal-escape portion may be positioned closer to the centrally-acting portion than the cell-penetrating sequence portion. From the viewpoint of achieving the effect of the invention more efficiently, the centrally-acting peptide derivative preferably has the cell-penetrating sequence portion at a position closer to the centrally-acting portion than the endosomal-escape portion, and more preferably has a PAS as the endosomal-escape portion at the N-terminal side of the cell-penetrating sequence portion.

### (Spacer)

In the centrally-acting peptide derivative, the centrally-acting portion may be linked to the cell-penetrating sequence portion or the endosomal-escape portion directly or via a spacer. When a spacer is positioned between the centrally-acting portion and the cell-penetrating sequence portion, the amino acid sequence of the spacer is not particularly restricted as long as it has a function to suppress a decrease or a loss of the activity of the centrally-acting portion. Generally, the cell-penetrating sequence portion is formed of basic amino acid residues. Therefore, when the centrally-acting portion includes an acidic amino acid residue, it is preferred to position a spacer having a sequence formed of one or more (for example, 1 to 10, preferably 2 to 6) neutral amino acid residues, such as glycine, in order to suppress a decrease or a loss of the activity of the centrally-acting portion caused by an interaction between the cell-penetrating sequence portion and the centrally-acting portion.

The centrally-acting peptide derivative may be subjected various modification according to the intended purposes. Examples of the modification include amino gorup modification (such as biotinylation, myristoylation, palmitoylation, acetylation and maleimidation), carboxy group modification (such as amidation and esterification), thiol group modification (such as farnesylation, geranylation, methylation and palmitoylation), hydroxy group modification (such as phosphorylation, sulfation, octanoylation, palmitoylation and palmitoleoylation, fluorescence labelling (such as FITC, FAM, rhodamine, BODIPY, NBD and MCA), pegylation, and introduciton of unnatural amino acids, D-amino acids or the like. The modification may be performed on any of the centrally-acting portion, cell-penetrating sequence portion, the endosomal-escape portion or the spacer of the centrally-acting peptide derivative.

Each of the amino acid residues that constitute the centrally-acting peptide derivative may be either L-form or D-form, as long as the effect of the invention is achieved. The method of obtaining the centrally-acting peptide derivative is not particularly restricted, and may be extracted from a living body or a natural substance, or may be prepared by a genetic engineering process or an organic synthetic process.

In an embodiment, the centrally-acting peptide derivative is formed of an amino acid sequence in which an amino acid sequence of PAS, an amino acid sequence of CPP, an amino acid sequence of a spacer as necessary, and an amino acid sequence of the centrally-acting portion are positioned in this order from the N-terminal side, where PAS is selected from FFLIPKG, LILIG, FFG, FFFFG or FFFFFFG, CPP is selected from oligoarginine (Rn, n=6-12), and the spacer is selected from glycine residues (Gn, n=2-6).

When the centrally-acting portion of the centrally-acting peptide derivative is an amino acid sequence, the total number of amino acid residues of the centrally-acting peptide derivative is not particularly restricted as long as the effect of the invention is achieved, and may be selected according to the intended purposes. For example, the total number of amino acid residues of the centrally-acting peptide derivative may be 80 or less, preferably 70 or less, more preferably 60 or less. For example, the total number of amino acid residues of the centrally-acting peptide derivative may be 35 or more, preferably 40 or more, more preferably 45 or more.

### <Pharmaceutical composition>

The pharmaceutical composition of the invention includes a centrally-acting peptide derivative having a centrally-acting portion, a cell-penetrating sequence portion and an endosomal-escape portion as an active ingredient. By including a centrally-acting peptide derivative as an active ingredient, the pharmaceutical composition can be rapidly delivered to the brain and efficiently exert a pharmacological effect. Further, since the pharmaceutical composition can be administered is a less invasive manner, it is useful as a drug that needs to be taken on a daily basis at home. Therefore, a nasal formulation is a preferred form of the pharmaceutical composition.

The neuropsychiatric disorder, which is a therapeutic objective of the pharmaceutical composition, is not particularly restricted as long as a therapeutic effect is exerted by the action of the centrally-acting portion of the centrally-acting peptide derivative on the central nervous system. Examples of the neuropsychiatric disorder include depression, learning disorder, anxiety, eating disorder, cognition disorder, high blood pressure, sleeping disorder and epilepsia.

Specific examples of the pharmaceutical composition include an anti-depression agent, a learning disorder-alleviating agent, an anti-anxiety agent, a feeding suppression agent, a cognitive disorder-alleviating agent, a blood-pressure-lowering agent, an analgesic agent, a sleep-inducing agent, and an anti-epileptic agent. The type of the centrally-acting portion, the cell-penetrating sequence portion and the endosomal-escape portion of the centrally-acting peptide derivative may be selected according to the therapeutic objective. For example, a pharmaceutical composition including a centrally-acting peptide derivative having a peptide portion derived from GLP-2 as an active ingredient is effective as an anti-depression agent. Further, since GLP-2 exerts a blood pressure-lowering activity, the pharmaceutical composition including a centrally-acting peptide derivative having a peptide portion derived from GLP-2 as an active ingredient is considered to be particularly effective for a patient with depression due to strong pressure and high blood pressure in combination. A pharmaceutical composition including a centrally-acting peptide derivative having a peptide portion derived from GLP-1 or NmU as an active ingredient is effective as a learning disorder-alleviating agent.

The details and the preferred embodiments of the centrally-acting peptide derivative included in the pharmaceutical composition are as described above.

The method of using the pharmaceutical composition is not particularly restricted, and may be any of intranasal administration, oral administration, intravenous administration and the like. In view of adaptability to daily administration, intranasal administration and oral administration are preferred. In view of adaptability to delivery to the brain, intranasal administration is more preferred. The pharmaceutical composition may include a component other than the centrally-acting peptide derivative. Examples of the component other than the centrally-acting peptide derivative include a medium and a formulation additive that are used for the preparation of a pharmaceutical composition. Specific examples of the formulation additive include a diluent, a disintegrant, a binder, a lubricant, a surfactant, a buffer, a solubilizing agent, a stabilizer, a tonicity agent, a suspending agent, an emulcifier, a solvent, a thickner, a mucolytic agent, a humectant and a preservative. The dosage amount of the pharmaceutical composition may be selected according to the type of disease, the symptomatic state, weight or age of the patient, the administration form and the like.

### <Nasal formulation>

The nasal formulation of the inveniton includes a centrally-acting peptide derivative having a centrally-acting portion, a cell-penetrating sequence portion and an endosomal-escape portion as an active ingredient. By including a centrally-acting peptide derivative as an active ingredient, the nasal formulation can be rapidly delivered to the brain and efficiently exert a pharmacological effect. Further, since the nasal formulation can be administered is a less invasive manner, it is useful as a drug that needs to be taken on a daily basis at home. Therefore, a nasal formulation is a preferred form of the pharmaceutical composition.

The deails and the preferred embodiments of the centrally-acting peptide derivative included in the nasal formulation are as described above. The nasal formulation may include a component other than the centrally-acting peptide derivative. Examples of the component other than the centrally-acting peptide derivative include a medium and a formulation additive that may be used for the preparation of the pharmaceutical composition as descried above.

The embodiments of the invention include a use of the pharmaceutical composition including a centrally-acting peptide derivative as an active ingredient, as described above, for intranasal administration. The details and the preferred embodiments of the centrally-acting peptide derivative for the use are as described above.

The embodiments of the invention include a method of stabilizing a centrally-acting peptide, the method including adding a cell-penetrating sequence portion and an endosomal-escape portion to the centrally-acting peptide. The details and the preferred embodiments of the cell-penetrating sequence portion, the endosomal-escape portion and the centrally-acting peptide in the method are as described above.

### [Examples]

The invention are explained in further detail by referring to the following examples. The materials, amounts, rates, procedures and the like may be modified without departing from the scope of the invention. Therefore, the scope of the invention should not be construed in a restricted manner by the examples as described below.

Evaluation on anti-depression activity of centrally-acting peptide peptide derivative

### <Example 1>

A derivative of GLP-2 (GLP-2 derivative) having an amino acid sequence of FITC-FFLIPKG-(R)₈-(G)₂-HADGSFSDEMNTILDNLAARDFINWLIQTKITD was prepared by an ordinary method as a centrally-acting peptide derivative. The GLP-2 derivative was primarily dissolved in DMSO and diluted with phosphate-buffered saline (PBS) to adjust the final concentration of DMSO to 16%, thereby preparing a DMSO solution of GLP-2 derivative.

The anti-depression activity of the GLP-2 derivative was evaluated by a forced-swim test on rats using the DMSO solution of GLP-2 derivative. The forced-swim test was performed in accordance with the following procedure.

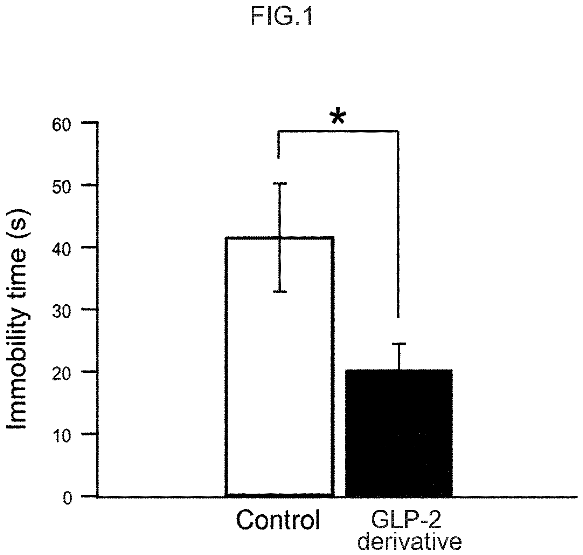

One hour before the test, rats (8-10 week old, male, n=6) were transferred to the laboratory for habituation. The rats were intranasally administered with the DMSO solution of GLP-2 derivative by 18 µg/day for two days, 30 minutes before the test. The rats were allowed to swim for 15 minutes, one by one in a transparent plastic cylinder with a diameter of 18 cm and a height of 50 cm filled with water of 25±1°C to a height of 30 cm. During the swimming, the immobility time (s) was measured. The rats after the test were wiped with a Kimtowel and placed back to a cage. The test was performed for consecutive 2 days, day 1 as a preliminary test and day 2 as a main test. As a control, rats (8-10 week old, male, n=6) were intranasally administered with a solvent without GLP-2 derivative by 10 µL and subjected to a forced-swim test in the same manner. The results are shown in Table 1 and Fig. 1. The error bar represents mean±SE (n=6), and the statistical processing was performed by Mann-Whitney test (*P<0.05).

### <Comparative Example 1>

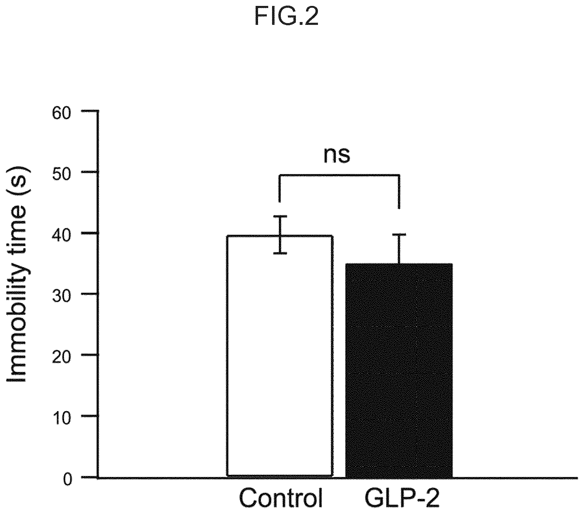

A peptide having an amino acid sequence of HADGSFSDEMNTILDNLAARDFINWLIQTKITD, i.e., an amino acid sequence of GLP-2 alone without a cell-penetrating sequence portion and an endosomal-escape portion, was prepared by an ordinary method, and a DMSO solution of GLP-2 (16%) was prepared in the same manner as Example 1. The forced-swim test was performed in the same manner as Example 1, except that the rats (8-10 week old, male, n=6) were intranasally administered with the DMSO solution of GLP-2 by 18 µg/day. The results are shown in Table 1 and Fig. 2. The error bar represents mean±SE (n=6), and the statistical processing was performed by Mann-Whitney test (ns refers to not significant).

**Table 1**

| Immobility time (s) | | | |
|---|---|---|---|
| Example 1 | | Comparative Example 1 | |
| Control | GLP-2 derivative | Control | GLP-2 |
| 55 | 36 | 48 | 45 |
| 22 | 13 | 43 | 41 |
| 45 | 8 | 35 | 30 |
| 25 | 25 | 47 | 33 |
| 76 | 16 | 30 | 46 |
| 26 | 24 | 35 | 15 |

### <Example 2>

The state of distribution of GLP-2 derivative in olfactory bulb, infralimbic cortex, basolateral amygdala, central amygdala), medial amygdala, paraventricular hypothalamic nucleus, dorsomedial hypothalamic nucleus, hippocampus and parabrachial nucleus of rats was evaluated by the following process. The rats were intranasally administered with a DMSO solution of GLP-2 derivative (16%) by18 µg in the same manner as Example 1.

Thirty minutes after the intranasal administration to a rat with a DMSO solution of GLP-2 derivative (16%) as prepared in the same manner as Example 1, the rat was formalin-fixed to prepare a brain specimen. A section (30 µm) was obtained from the brain specimen with a cryostat (Leica Microsystems, CM1560S), and the section was observed with a fluorescence microscope (Keyence Corporation, BZ-9000). As a control, a brain specimen of a rat intranasally administered with a solvent without GLP-2 was prepared and observed in the same manner. The results are shown in Table 1. The error bar represents mean ± SE (n = 6), and the statistical processing was performed by Mann-Whitney test (*P<0.05, **P<0.01).

### <Example 3>

A GLP-2 derivative having an amino acid sequence of FFLIPKG-(R)₈-(G)₂-HADGSFSDEMNTILDNLAARDFINWLIQTKITD was prepared as a centrally-acting peptide derivative. This DLP-2 derivative is different from the GLP-2 derivative prepared in Example 1 in that FITC is not added thereto. The GLP-2 derivative is primarily dissolved in DMSO and diluted with phosphate buffer (PBS) in order to adjust the final concentration of DMSO to 16%, thereby preparing a DMSO solution of GLP-2 derivative.

The anti-depression activity of the GLP-2 derivative was evaluated by a forced-swim test on a mouse. The test was performed by the following process.

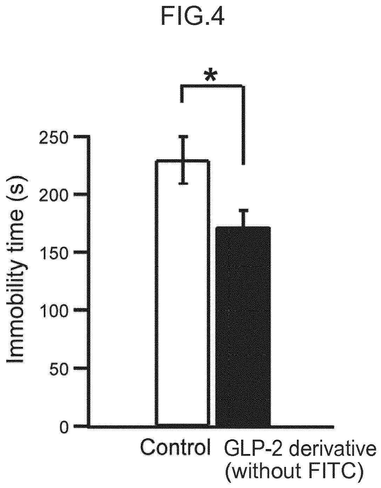

Twenty-four hours before the test, ddY mice (5-7 week old, male, n=12) were subjected to a preliminary swimming for 15 minutes. The mice were administered GLP-2 derivative by 3.6 µg with the DMSO solution, on the day before the test and on the day for the test, respectively. Then, the test was performed in the same manner as Example 1, and the immobility time (s) was measured. As a control, mice (5-7 week old, male, n=12) were administered with a solution without GLP-2 derivative were subjected to a forced-swim test in the same manner. The results are shown in Table 2 and Fig. 4. The error bar represents mean±SE (n=12), and the statistical processing was performed by Mann-Whitney test (*P<0.05).

**Table 2**

| Immobility time (s) | |
|---|---|
| Control | GLP-2 derivative |
| 205 | 163 |
| 263 | 65 |
| 216 | 217 |
| 218 | 147 |
| 278 | 185 |
| 47 | 152 |
| 276 | 154 |
| 175 | 151 |
| 239 | 231 |
| 280 | 218 |
| 321 | 236 |
| 236 | 141 |

### <Example 4>

The anti-depression activity of the GLP-2 derivative was evaluated by a tail suspension test on a mouse, using the DMSO solution of GLP-2 derivative as prepared in Example 3. The test was performed by the following process.

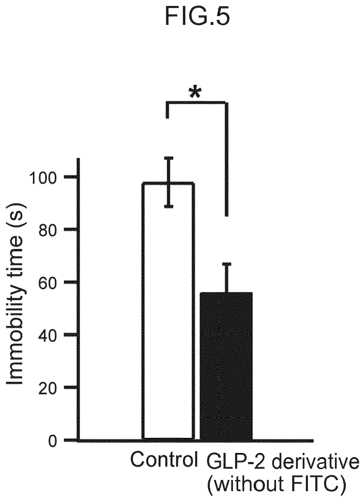

On the day before the test and on the day for the test, ddY mice (5-7 week old, male, n = 9) were administered with the GLP-derivative by 3.6 µg, respectively, with the DMSO solution. Then, the mice were suspended by the tail and the immobility time (s) was measured. As a control, mice (5-7 week old, male, n = 9) were intranasally administered with a solvent without GLP-2 derivative and subjected to a tail suspension test in the same manner. The results are shown in Table 3 and Fig. 5. The error bar represents mean±SE (n=9), and the statistical processing was performed by Mann-Whitney test (*P<0.05).

**Table 3**

| Immobility time (s) | |
|---|---|
| Control | GLP-2 derivative |
| 57 | 27 |
| 74 | 49 |
| 121 | 131 |
| 147 | 45 |
| 73 | 71 |
| 101 | 34 |
| 107 | 27 |
| 92 | 54 |
| 109 | 67 |

### <Example 5>

The anti-depression activity of the GLP-2 derivative was evaluated by a forced-swim test on a depression model rat using the DMSO solution of GLP-2 derivative as prepared in Example 3.

As the depression model mouse, ddY mice (5-7 week old, male, n=11) were administered with adrenocorticotropic hormone (ACTH) to induce an enhanced state of hypothalamic-pituitary-adrenal axis (HPA system). The administration of ACTH was performed by 0.45mg/kg/day for consecutive 14 days.

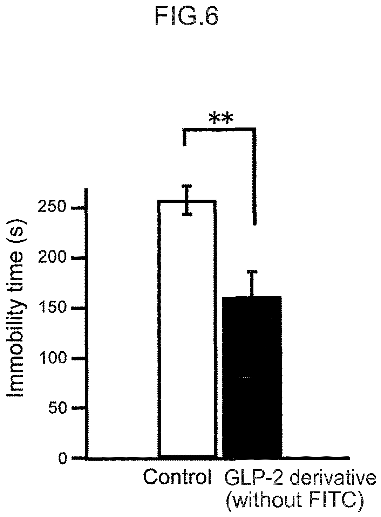

The forced-swim test was performed in the same manner as Example 3 and the immobility time (s) was measured. The mice were intranasally administered with GLP-2 derivative by 3.6 µg as a DMSO solution of GLP-2 derivative, each day for consecutive 6 days until the day for the test. As a control, mice (5-7 week old, male, n=11) were administered with a solvent without GLP-2 derivative by 4 µL each day for consecutive 6 days and subjected to a forced-swim test in the same manner. The results are shown in Table 4 and Fig. 6. The error bar represents mean±SE (n=11), and the statistical processing was performed by Mann-Whitney test (*P<0.01).

**Table 4**

| Immobility time (s) | |
|---|---|
| Control | GLP-2 derivative |
| 193 | 25 |
| 214 | 113 |
| 251 | 268 |
| 307 | 149 |
| 177 | 287 |
| 265 | 58 |
| 289 | 207 |
| 310 | 192 |
| 252 | 151 |
| 316 | 221 |
| 264 | 96 |

### Evaluation on learning disorder-alleviating activity of centrally-acting peptide derivative

### <Example 6>

A derivative of GLP-1 (7-36 amide) having an amino acid sequence of FFLIPKG-(R)₈-(G)₂-HAEGTFTSDVSSYLEGQAAKEFIAWLVKGR-NH₂ was prepared by an ordinary method as a centrally-acting peptide derivative. The GLP-1 derivative was primarily dissolved in FOS 12 (n-dodecylphosphocholine), and diluted with PBS to adjust the final concentration of FOS 12 to 1%, thereby preparing a FOS 12 solution of GLP-1 derivative.

The learning disorder-alleviating activity of the GLP-1 derivative was evaluated by a Y-maze test on a learning disorder-induced mouse, using the FOS 12 solution of GLP-1 derivative.

As the learning disorder-induced mouse, ddY mice (5-7 week old, male, n=5) were administered with lipopolysaccharide (LPS) by 10 µg at three days before the test. Fifteen minutes before the administration of LPS, the mice were administered with GLP-1 derivative by 3 µg with the FOS 12 solution of GLP-1 derivative.

The Y-maze test was performed by using a device having three arms of the same size. The number of times that the mouse entered the different arm for consecutive three times was counted, and the result was divided by a number obtained by deducting 2 from the total number of times of entering the arm. The result was multiplied by 100, and used as a spontaneous alternation behavior (%).

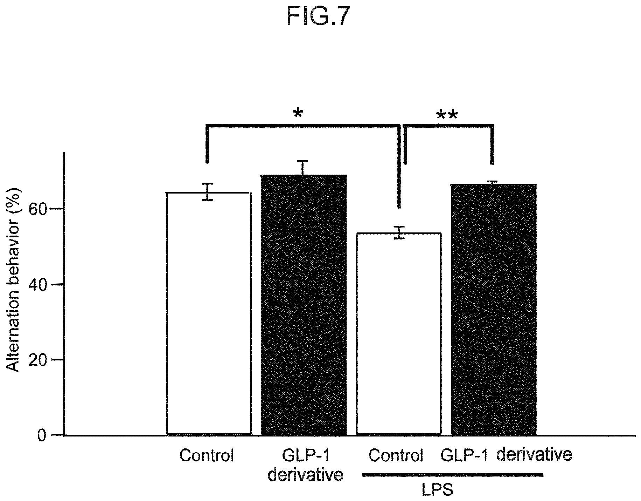

As a control, mice (5-7 week old, male, n =5 or 7), intranasally administered a solvent without GLP-1 derivative by 4 µL 15 minutes before the administration of LPS, were subjected to the same test as the above. Further, mice that were not administered with LPS (vehicle) were subjected to the same test as the above. The results are shown in Table 5 and Fig. 7. The error bar represents mean±SE (n=5 or 7), and the statistical processing was performed by Mann-Whitney test (*P<0.05, **P<0.01).

**Table 5**

| Alternation behavior (%) | | | |
|---|---|---|---|
| Vehicle | | LPS | |
| Control | GLP-1 derivative | Control | GLP-1 derivative |
| 65.7 | 72.2 | 52.0 | 66.7 |
| 60.0 | 70.3 | 56.5 | 66.7 |
| 66.7 | 78.6 | 48.6 | 67.9 |
| 59.6 | 67.6 | 54.8 | 67.4 |
| 61.5 | 56.4 | 56.8 | 64.3 |
| 76.0 | | | |
| 62.1 | | | |

### Evaluation on learning disorder-alleviating activity of centrally-acting peptide derivative

### <Example 7>

A derivative of NmU having an amino acid sequence of FFFFG-(R)₈-(G)₂-YKVNEYQGPVAPSGGFFLFRPRN-NH₂ was prepared by an ordinary method as a centrally-acting peptide derivative. The NmU derivative was primarily dissolved in FOS 12 and diluted with PBS to adjust the final concentration of FOS 12 to 5%, thereby preparing a FOS 12 solution of NmU derivative.

As the learning disorder-induced mouse, ddY mice (5-7 week old, male, n = 6) administered with lipopolysaccharide (LPS) by 10 µg three days before the test were used. Fifteen minutes before the administration of LPS, the mice were administered with NmU derivative by 3 µg with the FOS 12 solution of NmU derivative. A Y-maze test was performed in the same manner as Example 6, and a spontaneous alternation behavior (%) was calculated from the measurement results.

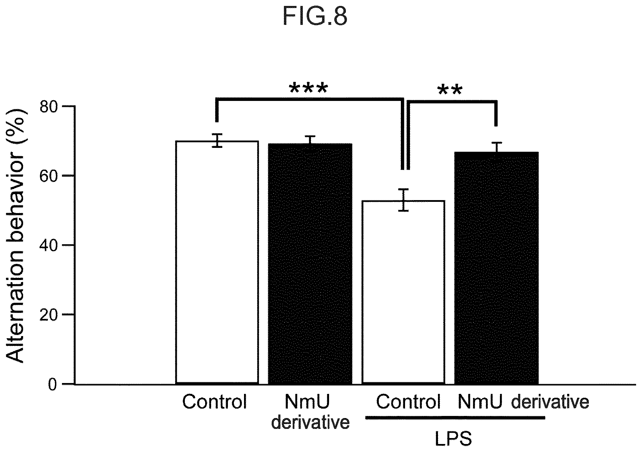

As a control, mice (5-7 week old, male, n = 6), which were intranasally administered a solvent without NmU derivative by 4 µL 15 minutes before the administration of LPS, were subjected to the same test as the above. Further, mice not administered with LPS (vehicle) were subjected to the same test as the above. The results are shown in Table 6 and Fig. 8. The error bar represents mean±SE (n=6), and the statistical processing was performed by Mann-Whitney test (*P<0.01, **P<0.001).

**Table 6**

| Alternation behavior (%) | | | |
|---|---|---|---|
| Vehicle | | LPS | |
| Control | NmU derivative | Control | NmU derivative |
| 67.7 | 69.0 | 68.2 | 66.7 |
| 66.7 | 63.9 | 52.1 | 60.0 |
| 79.1 | 67.8 | 50.8 | 74.1 |
| 69.2 | 78.8 | 48.9 | 69.4 |
| 68.6 | 65.9 | 49.0 | 58.6 |
| 69.6 | 70.5 | 48.9 | 72.4 |

### Evaluation on stability of centrally-acting peptide derivative

### <Example 8>

The GLP-2 and the GLP-2 derivative prepared in Example 3 were primarily dissolved in DMSO and diluted with PBS to adjust the final concentration of DMSO to 1%, respectively, thereby preparing samples.

The content of GLP-2 or GLP-2 derivative in each sample was adjusted to 12 ng/µL, and DPP-4 (dipeptidyl peptidase-4) was added to each sample.

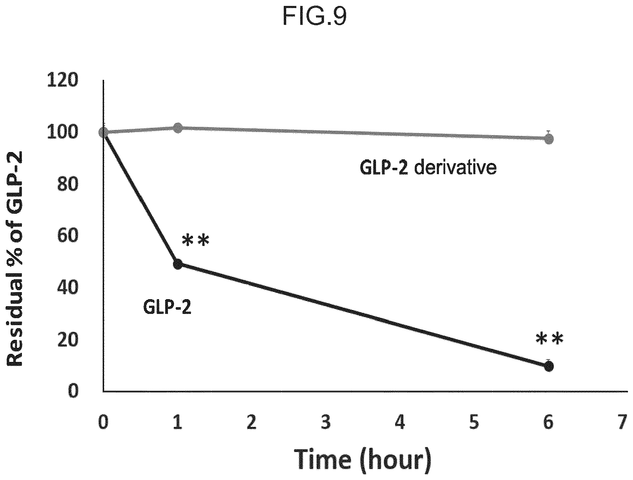

The samples were stored at 37 °C, and the content of the GLP-2 or GLP-2 derivative was measured at 1 hour and 6 hours after the preparation. The measured values were converted to a relative value as a residual ratio (%) based on the content of the GLP-2 or GLP-2 derivative immediately after the preparation as 100%. The results are shown in Fig. 9. The value shown in Fig. 9 represents mean ± SE (n = 3), and the statistical processing was performed by t-test (**P<0.001).

### Analysis on evaluation results

The results of Example 1 and Comparative Example 1 showed that the immobility time in the forced-swim test of the group administered with GLP-2 derivative, prepared by adding a cell-penetrating sequence portion and an endosome-escape portion to GLP-2, was significantly shorter than that of the control group, indicating a significant difference in the anti-depression activity between the groups. In contrast, no significant difference in the anti-depression activity was shown between the group administered with GLP-2, not added with a cell-penetrating sequence portion and an endosome-escape portion, and the control group. These results suggest that the anti-depression activity of GLP-2 is improved by adding a cell-penetrating sequence portion and an endosome-escape portion thereto.

The results of Example 2 show that the GLP-2 derivative widely distributes in the brain, indicating that the improvement in anti-depression activity, achieved by adding a cell-penetrating sequence portion and an endosomal-escape portion to GLP-2, is due to the enhanced distribution of GLP-2 in the brain.

The results of Example 3 and Example 4, in which the test object and the test method were changed, and Example 5, in which a treatment-resistive depression model was used, suggest that an effect of suppressing depression is obtained by the administration of GLP-2.

The results of Example 6 and Example 7, in which a Y-maze test was performed with GLP-1 and NmU as a centrally-acting peptide, suggest that the learning ability is significantly ameliorated by the administration of GLP-1 or NmU, while the learning ability of the LPS-administered group was lower than that of the vehicle group (not administered with LPS).

The results of Example 8, a stability test of a centrally-acting peptide derivative, showed that the GLP-2 derivative is more stable than GLP-2 alone with respect to DPP-4, which is an enzyme capable of decomposing GLP-2. These results suggest that the addition of a cell-penetrating sequence portion and an endosomal-escape portion to a centrally-acting portion suppress the decomposition of GLP-2 by DPP-4, thereby providing additional effects such as improved stability of a peptide and improved ability of migrating to the central nervous system.

### SEQUENCE LISTING

<110> Tokyo University of Science Foundation
<120> CENTRALLY-ACTING PEPITIDE DERIVATIVE AND MEDICAL COMPOSITION
<130> CO-F04124-00
<150> JP 2014-177974
   <151> 2014-09-02
<150> JP 2014-184436
   <151> 2014-09-10
<160> 90
<170> PatentIn version 3.5
<210> 1
   <211> 33
   <212> PRT
   <213> Homo Sapience
<220>
   <223> GLP-2
<400> 1
<210> 2
   <211> 30
   <212> PRT
   <213> Homo Sapience
<220>
   <223> GLP-1 (7-36)amide
<400> 2
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 3
   <211> 23
   <212> PRT
   <213> Porcine spinal cord
<220>
   <223> Neuromedin U
<400> 3
<210> 4
   <211> 19
   <212> PRT
   <213> Artificial sequence
<220>
   <223> Angiopep-5
<400> 4
<210> 5
   <211> 16
   <212> PRT
   <213> Artificial sequence
<220>
   <223> Antp
<400> 5
   Arg Gln Ile Lys Ile Trp Phe Gln Asn Arg Arg Met Lys Trp Lys Lys
   1               5                   10                  15
<210> 6
   <211> 11
   <212> PRT
   <213> Artificial sequence
<220>
   <223> Antp 6 Bac
<400> 6
<210> 7
   <211> 13
   <212> PRT
   <213> Artificial sequence
<220>
   <223> BR1
<400> 7
<210> 8
   <211> 17
   <212> PRT
   <213> Artificial sequence
<220>
   <223> BR2
<400> 8
<210> 9
   <211> 21
   <212> PRT
   <213> Artificial sequence
<220>
   <223> Buf IIb[BR3]
<400> 9
<210> 10
   <211> 5
   <212> PRT
   <213> Artificial sequence
<220>
   <223> SH-CPPP-2
<400> 10
   Lys Leu Pro Val Met
   1               5
<210> 11
   <211> 10
   <212> PRT
   <213> Artificial sequence
<220>
   <223> CyLoP-1
<400> 11
<210> 12
   <211> 17
   <212> PRT
   <213> Artificial sequence
<220>
   <223> Cys-Antp
<400> 12
<210> 13
   <211> 19
   <212> PRT
   <213> Artificial sequence
<220>
   <223> Cys-pVEC
<400> 13
<210> 14
   <211> 19
   <212> PRT
   <213> Artificial sequence
<220>
   <223> Cys-SAP
<400> 14
<210> 15
   <211> 19
   <212> PRT
   <213> Artificial sequence
<220>
   <223> Cys-SAPr
<400> 15
<210> 16
   <211> 12
   <212> PRT
   <213> Artificial sequence
<220>
   <223> Cys-TAT
<400> 16
<210> 17
   <211> 22
   <212> PRT
   <213> Artificial sequence
<220>
   <223> Cys-TP10
<400> 17
<210> 18
   <211> 23
   <212> PRT
   <213> Artificial sequence
<220>
   <223> Cys-TP10K
<400> 18
<210> 19
   <211> 21
   <212> PRT
   <213> Artificial sequence
<220>
   <223> FAK
<400> 19
<210> 20
   <211> 9
   <212> PRT
   <213> Artificial sequence
<220>
   <223> H9
<400> 20
<210> 21
   <211> 15
   <212> PRT
   <213> Artificial sequence
<220>
   <223> HC-[poly(K)]
<400> 21
   Gln Tyr Ile Lys Ala Asn Ser Lys Phe Ile Gly Ile Thr Glu Leu
   1 5 10 15
<210> 22
   <211> 22
   <212> PRT
   <213> Artificial sequence
<220>
   <223> hLF
<400> 22
<210> 23
   <211> 22
   <212> PRT
   <213> Artificial sequence
<220>
   <223> HR9
<400> 23
<210> 24
   <211> 32
   <212> PRT
   <213> Artificial sequence
<220>
   <223> IR9
<400> 24
   Gly Leu Phe Glu Ala Ile Glu Gly Phe Ile Glu Asn Gly Trp Glu Gly
   1               5                   10                  15
   Met Ile Asp Gly Trp Tyr Gly Arg Arg Arg Arg Arg Arg Arg Arg Arg
               20                  25                  30
<210> 25
   <211> 33
   <212> PRT
   <213> Artificial sequence
<220>
   <223> JNKI 1D TAT
<400> 25
<210> 26
   <211> 9
   <212> PRT
   <213> Artificial sequence
<220>
   <223> K9
<400> 26
<210> 27
   <211> 14
   <212> PRT
   <213> Artificial sequence
<220>
   <223> Low molecular weight protamine (LMWP)
<400> 27
<210> 28
   <211> 18
   <212> PRT
   <213> Artificial sequence
<220>
   <223> MAP
<400> 28
<210> 29
   <211> 14
   <212> PRT
   <213> Artificial sequence
<220>
   <223> Mitoparan
<400> 29
<210> 30
   <211> 25
   <212> PRT
   <213> Artificial sequence
<220>
   <223> m-N2-10-1 (MTS-C)
<400> 30
<210> 31
   <211> 25
   <212> PRT
   <213> Artificial sequence
<220>
   <223> m-N2-10-1 (MTS-N)
<400> 31
<210> 32
   <211> 23
   <212> PRT
   <213> Artificial sequence
<220>
   <223> m-N2-10-1 (NIV-C)
<400> 32
<210> 33
   <211> 23
   <212> PRT
   <213> Artificial sequence
<220>
   <223> m-N2-10-1 (NIV-N)
<400> 33
<210> 34
   <211> 18
   <212> PRT
   <213> Artificial sequence
<220>
   <223> m-N2-10-1 (OR-C)
<400> 34
<210> 35
   <211> 18
   <212> PRT
   <213> Artificial sequence
<220>
   <223> m-N2-10-1 (OR-N)
<400> 35
<210> 36
   <211> 31
   <212> PRT
   <213> Artificial sequence
<220>
   <223> MPG-8
<400> 36
   Ala Phe Leu Gly Trp Leu Gly Ala Trp Gly Thr Met Gly Trp Ser Pro
   1               5                   10                  15
   Lys Lys Lys Arg Lys Cys Tyr Ser Thr Glu Ala Met Ile Asp Glu
               20                  25                  30
<210> 37
   <211> 32
   <212> PRT
   <213> Artificial sequence
<220>
   <223> MPG***α***
<400> 37
   Gly Ala Leu Phe Leu Ala Ala Ala Leu Ser Leu Met Gly Leu Trp Ser
   1               5                   10                  15
   Gln Pro Lys Lys Lys Arg Lys Val Asn His Cys His Cys His Ser His
               20                  25                  30
<210> 38
   <211> 32
   <212> PRT
   <213> Artificial sequence
<220>
   <223> MPG***β***
<400> 38
   Gly Ala Leu Phe Leu Gly Ala Ala Leu Ser Leu Met Gly Leu Trp Ser
   1               5                   10                  15
   Gln Pro Lys Lys Lys Arg Lys Val Asn His Cys His Cys His Ser His
               20                  25                  30
<210> 39
   <211> 14
   <212> PRT
   <213> Artificial sequence
<220>
   <223> NrTP1
<400> 39
<210> 40
   <211> 15
   <212> PRT
   <213> Artificial sequence
<220>
   <223> NrTP2
<400> 40
<210> 41
   <211> 14
   <212> PRT
   <213> Artificial sequence
<220>
   <223> NrTP5
<400> 41
<210> 42
   <211> 14
   <212> PRT
   <213> Artificial sequence
<220>
   <223> NrTP6
<400> 42
<210> 43
   <211> 14
   <212> PRT
   <213> Artificial sequence
<220>
   <223> NrTP7
<400> 43
<210> 44
   <211> 27
   <212> PRT
   <213> Artificial sequence
<220>
   <223> MPG HIV-gp41/SV40 T-antigen
<400> 44
<210> 45
   <211> 14
   <212> PRT
   <213> Artificial sequence
<220>
   <223> NrTP8
<400> 45
<210> 46
   <211> 19
   <212> PRT
   <213> Artificial sequence
<220>
   <223> PAMAM-PEG-Angiopep-2
<400> 46
<210> 47
   <211> 12
   <212> PRT
   <213> Artificial sequence
<220>
   <223> PEI-TAT
<400> 47
<210> 48
   <211> 16
   <212> PRT
   <213> Artificial sequence
<220>
   <223> Penetratin (43-58)
<400> 48
   Arg Gln Ile Lys Ile Trp Phe Gln Asn Arg Arg Met Lys Trp Lys Lys
   1               5                   10                  15
<210> 49
   <211> 17
   <212> PRT
   <213> Artificial sequence
<220>
   <223> Penetratin
<400> 49
<210> 50
   <211> 21
   <212> PRT
   <213> Artificial sequence
<220>
   <223> Pep-1 HIV-reverse transcriptase/SV40 T-antigen
<400> 50
<210> 51
   <211> 21
   <212> PRT
   <213> Artificial sequence
<220>
   <223> Pep-1
<400> 51
<210> 52
   <211> 28
   <212> PRT
   <213> Artificial sequence
<220>
   <223> pepM residues 45-72 of DENV-2 C protein
<400> 52
<210> 53
   <211> 35
   <212> PRT
   <213> Artificial sequence
<220>
   <223> pepR residues 67-100 of DENV-2 C protein
<400> 53
<210> 54
   <211> 13
   <212> PRT
   <213> Artificial sequence
<220>
   <223> peptide derived from protamine
<400> 54
<210> 55
   <211> 18
   <212> PRT
   <213> Artificial sequence
<220>
   <223> Peptide vascular endothelial cadherin (pVEC)
<400> 55
<210> 56
   <211> 21
   <212> PRT
   <213> Artificial sequence
<220>
   <223> PF14
<400> 56
<210> 57
   <211> 16
   <212> PRT
   <213> Artificial sequence
<220>
   <223> PR9
<400> 57
   Phe Phe Leu Ile Pro Lys Gly Arg Arg Arg Arg Arg Arg Arg Arg Arg
   1 5 10 15
<210> 58
   <211> 15
   <212> PRT
   <213> Artificial sequence
<220>
   <223> protamine
<400> 58
   Val Ser Arg Arg Arg Arg Arg Arg Gly Gly Arg Arg Arg Arg Lys
   1 5 10 15
<210> 59
   <211> 26
   <212> PRT
   <213> Artificial sequence
<220>
   <223> PYC36L-TAT
<400> 59
<210> 60
   <211> 9
   <212> PRT
   <213> Artificial sequence
<220>
   <223> R6W3 Based on penetratin
<400> 60
<210> 61
   <211> 31
   <212> PRT
   <213> Artificial sequence
<220>
   <223> R9-hLF
<400> 61
   Arg Arg Arg Arg Arg Arg Arg Arg Arg Lys Cys Phe Gln Trp Gln Arg
   1               5                   10                  15
   Asn Met Arg Lys Val Arg Gly Pro Pro Val Ser Cys Ile Lys Arg
               20                  25                  30
<210> 62
   <211> 9
   <212> PRT
   <213> Artificial sequence
<220>
   <223> RA9
<400> 62
<210> 63
   <211> 9
   <212> PRT
   <213> Artificial sequence
<220>
   <223> RH9
<400> 63
<210> 64
   <211> 9
   <212> PRT
   <213> Artificial sequence
<220>
   <223> RL9
<400> 64
<210> 65
   <211> 38
   <212> PRT
   <213> Artificial sequence
<220>
   <223> RVG-9R
<400> 65
<210> 66
   <211> 9
   <212> PRT
   <213> Artificial sequence
<220>
   <223> RW9
<400> 66
<210> 67
   <211> 9
   <212> PRT
   <213> Artificial sequence
<220>
   <223> S8
<400> 67
<210> 68
   <211> 18
   <212> PRT
   <213> Artificial sequence
<220>
   <223> SAP
<400> 68
<210> 69
   <211> 18
   <212> PRT
   <213> Artificial sequence
<220>
   <223> SAPr
<400> 69
<210> 70
   <211> 18
   <212> PRT
   <213> Artificial sequence
<220>
   <223> SynB1
<400> 70
<210> 71
   <211> 10
   <212> PRT
   <213> Artificial sequence
<220>
   <223> SynB3
<400> 71
<210> 72
   <211> 7
   <212> PRT
   <213> Artificial sequence
<220>
   <223> Xentry
<400> 72
<210> 73
   <211> 22
   <212> PRT
   <213> Artificial sequence
<220>
   <223> YARA
<400> 73
<210> 74
   <211> 11
   <212> PRT
   <213> Artificial sequence
<220>
   <223> TAT (47-57)
<400> 74
<210> 75
   <211> 13
   <212> PRT
   <213> Artificial sequence
<220>
   <223> TAT (48-60)
<400> 75
<210> 76
   <211> 9
   <212> PRT
   <213> Artificial sequence
<220>
   <223> TAT (49-57)
<400> 76
<210> 77
   <211> 11
   <212> PRT
   <213> Artificial sequence
<220>
   <223> TAT-D
<400> 77
<210> 78
   <211> 11
   <212> PRT
   <213> Artificial sequence
<220>
   <223> TAT-L
<400> 78
<210> 79
   <211> 11
   <212> PRT
   <213> Artificial sequence
<220>
   <223> TAT-PEG-Cholesterol
<400> 79
<210> 80
   <211> 21
   <212> PRT
   <213> Artificial sequence
<220>
   <223> TP-10
<400> 80
<210> 81
   <211> 22
   <212> PRT
   <213> Artificial sequence
<220>
   <223> TP-10K
<400> 81
<210> 82
   <211> 27
   <212> PRT
   <213> Artificial sequence
<220>
   <223> Transportan
<400> 82
<210> 83
   <211> 20
   <212> PRT
   <213> Artificial sequence
<220>
   <223> CAD-2
<400> 83
<210> 84
   <211> 22
   <212> PRT
   <213> Artificial sequence
<220>
   <223> PF6
<400> 84
<210> 85
   <211> 11
   <212> PRT
   <213> Artificial sequence
<220>
   <223> polyarginine derivative
<400> 85
<210> 86
   <211> 7
   <212> PRT
   <213> Artificial sequence
<220>
   <223> PAS
<400> 86
<210> 87
   <211> 5
   <212> PRT
   <213> Artificial sequence
<220>
   <223> PAS
<400> 87
<210> 88
   <211> 3
   <212> PRT
   <213> Artificial sequence
<220>
   <223> PAS
<400> 88
<210> 89
   <211> 5
   <212> PRT
   <213> Artificial sequence
<220>
   <223> PAS
<400> 89
<210> 90
   <211> 7
   <212> PRT
   <213> Artificial sequence
<220>
   <223> PAS
<400> 90

## Claims

1. A nasal formulation comprising as an active ingredient a centrally-acting peptide derivative, said peptide derivative comprising a portion that acts on a central nervous system, a cell-penetrating sequence portion in which half or more of the total amino acid residues are basic amino acid residues, and an endosomal-escape portion selected from the group consisting of FFLIPKG, LILIG, FFG, FFFFG and FFFFFFG for use in therapy, wherein the formulation is administered nasally.

2. The nasal formulation according to claim 1, wherein the portion that acts on a central nervous system is an amino acid sequence portion that is derived from a peptide.

3. The nasal formulation according to claim 2, wherein the amino acid sequence portion is derived from a peptide that is GLP-1, GLP-2, neuromedin U, an opioid peptide, oxytocin, leptin, orexin, neuropeptide Y or insulin.

4. The nasal formulation according to claim 2 or claim 3, wherein the amino acid sequence portion is derived from a peptide selected from the following (a1), (a2), (a3) or (b):
(a1) a peptide comprising an amino acid sequence represented by His-Ala-Asp-Gly-Ser-Phe-Ser-Asp-Glu-Met-Asn-Thr-Ile-Leu-Asp-Asn-Leu-Ala-Ala-A rg-Asp-Phe-Ile-Asn-Trp-Leu-Ile-Gln-Thr-Lys-Ile-Thr-Asp (sequence number 1);
(a2) a peptide comprising an amino acid sequences represented by His-Ala-Glu-Gly-Thr-Phe-Thr-Ser-Asp-Val-Ser-Ser-Tyr-Leu-Glu-Gly-Gln-Ala-Ala-Lys -Glu-Phe-Ile-Ala-Trp-Leu-Val-Lys-Gly-Arg-NH₂ (sequence number 2);
(a3) a peptide comprising an amino acid sequence represented by Tyr-Lys-Val-Asn-Glu-Tyr-Gln-Gly-Pro-Val-Ala-Pro-Ser-Gly-Gly-Phe-Phe-Leu-Phe-Arg -Pro-Arg-Asn-NH₂ (neuromedin U, sequence number 3); and
(b) a peptide comprising an amino acid sequence represented by any of (a1) to (a3) in which one or more amino acid residues are deleted, replaced or added, the peptide having a centrally-acting activity.

5. The nasal formulation according to any one of claim 1 to claim 4, for use in the treatment of a neuropsychiatric disorder.

6. The nasal formulation according to any one of claim 1 to claim 4, for use in the treatment of depression or a learning disorder.

7. A pharmaceutical composition for use in therapy, wherein the formulation is administered nasally, comprising the nasal formulation according to any one of claim 1 to claim 4 as an active ingredient.

8. The pharmaceutical composition for use according to claim 7, for use in the treatment of a neuropsychiatric disorder.

9. The pharmaceutical composition for use according to claim 7, for use in the treatment of depression or a learning disorder.

10. A pharmaceutical composition comprising the centrally-acting peptide derivative according to any one of claim 1 to claim 4 as an active ingredient, wherein the pharmaceutical composition is for intranasal administration.

11. The nasal formulation for use according to claim 1, wherein the portion that acts on a central nervous system is selected from the group consisting of GLP-1, GLP-2 and neuromedin U, the cell-penetrating sequence portion is selected from an oligoarginine represented by Rn where n is from 6 to 12.

## Patentansprüche

1. Formulierung zur nasalen Verabreichung, umfassend als einen Wirkstoff, ein zentral wirkendes Peptidderivat, wobei das Peptidderivat einen Teil umfasst, der auf ein zentrales Nervensystem einwirkt, einen zellpenetrierenden Sequenzteil, in dem die Hälfte oder mehr der gesamten Aminosäurereste basische Aminosäurereste sind, und einen endosomal entweichenden Teil, ausgewählt aus der Gruppe, bestehend aus FFLIPKG, LILIG, FFG, FFFFG und FFFFFFG zur Verwendung in der Therapie, wobei die Formulierung nasal verabreicht wird.

2. Formulierung zur nasalen Verabreichung nach Anspruch 1, wobei der Teil, der auf ein zentrales Nervensystem einwirkt, ein Aminosäuresequenzteil ist, der von einem Peptid abgeleitet ist.

3. Formulierung zur nasalen Verabreichung nach Anspruch 2, wobei der Aminosäuresequenzteil von einem Peptid abgeleitet ist, das GLP-1, GLP-2, Neuromedin U, ein Opioidpeptid, Oxytocin, Leptin, Orexin, Neuropeptid Y oder Insulin ist.

4. Formulierung zur nasalen Verabreichung nach Anspruch 2 oder Anspruch 3, wobei der Aminosäuresequenzteil von einem Peptid abgeleitet ist, das aus den Folgenden (a1), (a2), (a3) oder (b) ausgewählt ist:
(a1) ein Peptid, enthaltend eine Aminosäuresequenz, die durch His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp (Sequenznummer 1) dargestellt ist;
(a2) ein Peptid, enthaltend eine Aminosäuresequenz, die durch His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-NH₂ (Sequenznummer 2) dargestellt ist;
(a3) ein Peptid, enthaltend eine Aminosäuresequenz, die durch Tyr-Lys-Val-Asn-Glu-Tyr-Gln-Gly-Pro-Val-Ala-Pro-Ser-Gly-Gly-Phe-Phe-Leu-Phe- Arg-Pro-Arg-Asn-NH₂ (Neuromedin U, Sequenznummer 3) dargestellt ist; und
(b) ein Peptid, das eine Aminosäuresequenz enthält, die durch eines aus (a1) bis (a3) dargestellt ist, in der ein oder mehrere Aminosäurereste deletiert, ersetzt oder hinzugefügt sind, wobei das Peptid eine zentral wirkende Aktivität aufweist.

5. Formulierung zur nasalen Verabreichung nach einem der Ansprüche 1 bis 4 zur Verwendung bei der Behandlung einer neuropsychiatrischen Störung.

6. Formulierung zur nasalen Verabreichung nach einem der Ansprüche 1 bis 4, zur Anwendung bei der Behandlung von Depressionen oder einer Lernstörung.

7. Pharmazeutische Zusammensetzung zur Verwendung in der Therapie, wobei die Formulierung nasal verabreicht wird, enthaltend die nasale Formulierung nach einem der Ansprüche 1 bis 4 als Wirkstoff.

8. Pharmazeutische Zusammensetzung zur Verwendung nach Anspruch 7, zur Verwendung bei der Behandlung einer neuropsychiatrischen Störung.

9. Pharmazeutische Zusammensetzung zur Verwendung nach Anspruch 7, zur Verwendung bei der Behandlung von Depressionen oder einer Lernstörung.

10. Pharmazeutische Zusammensetzung, enthaltend das zentral wirkende Peptidderivat nach einem der Ansprüche 1 bis 4 als Wirkstoff, wobei die pharmazeutische Zusammensetzung zur intranasalen Verabreichung bestimmt ist.

11. Formulierung zur nasalen Verabreichung zur Verwendung nach Anspruch 1, wobei der Teil, der auf ein zentrales Nervensystem einwirkt, ausgewählt ist aus der Gruppe, bestehend aus GLP-1, GLP-2 und Neuromedin U, der zellpenetrierende Sequenzteil ausgewählt ist aus einem Oligoarginin, dargestellt durch Rn, worin n 6 bis 12 ist.

## Revendications

1. Formulation nasale comprenant comme principe actif un dérivé peptidique agissant centralement, ledit dérivé peptidique comprenant une partie qui agit sur un système nerveux central, une partie de séquence pénétrant la cellule dans laquelle la moitié ou plus des résidus d'acide aminé totaux sont des résidus d'acide aminé basiques, et une partie d'échappement endosomal choisie dans le groupe constitué par FFLIPKG, LILIG, FFG, FFFFG et FFFFFFG destinée à être utilisée en thérapie, la formulation étant administrée par voie nasale.

2. Formulation nasale selon la revendication 1, dans laquelle la partie qui agit sur un système nerveux central est une partie de séquence d'acides aminés qui est dérivée d'un peptide.

3. Formulation nasale selon la revendication 2, dans laquelle la partie de séquence d'acides aminés est dérivée d'un peptide qui est GLP-1, GLP-2, la neuromédine U, un peptide opioïde, l'oxytocine, la leptine, l'orexine, le neuropeptide Y ou l'insuline.

4. Formulation nasale selon la revendication 2 ou la revendication 3, dans laquelle la partie de séquence d'acides aminés est dérivée d'un peptide choisi parmi les (a1), (a2), (a3) ou (b) suivants :
(al) un peptide comprenant une séquence d'acides aminés représentée par His-Ala-Asp-Gly-Ser-Phe-Ser-Asp-Glu-Met-Asn-Thr-Ile-Leu-Asp-Asn-Leu-Ala-Ala-Arg-Asp-Phe-Ile-Asn-Trp-Leu-Ile-Gln-Thr-Lys-Ile-Thr-Asp (séquence numéro 1)
(a2) un peptide comprenant une séquence d'acides aminés représentée par His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-NH₂ (séquence numéro 2)
(a3) un peptide comprenant une séquence d'acides aminés représentée par Tyr-Lys-Val-Asn-Glu-Tyr-Gln-Gly-Pro-Val-Ala-Pro-Ser-Gly-Gly-Phe-Phe-Leu-Phe- Arg-Pro-Arg-Asn-NH₂ (neuromédine U, séquence numéro 3) ; et
(b) un peptide comprenant une séquence d'acides aminés représentée par l'un quelconque de (a1) à (a3) dans lequel un ou plusieurs résidus d'acide aminé sont délétés, remplacés ou ajoutés, le peptide ayant une activité agissant centralement.

5. Formulation nasale selon l'une quelconque de la revendication 1 à la revendication 4, destinée à être utilisée dans le traitement d'un trouble neuropsychiatrique.

6. Formulation nasale selon l'une quelconque de la revendication 1 à la revendication 4, destinée à être utilisée dans le traitement de la dépression ou d'un trouble de l'apprentissage.

7. Composition pharmaceutique destinée à être utilisé en thérapie, dans laquelle la formulation est administrée par voie nasale, comprenant la formulation nasale selon l'une quelconque de la revendication 1 à la revendication 4 comme principe actif.

8. Composition pharmaceutique destinée à être utilisée selon la revendication 7, destinée à être utilisée dans le traitement d'un trouble neuropsychiatrique.

9. Composition pharmaceutique destinée à être utilisée selon la revendication 7, destinée à être utilisée dans le traitement de la dépression ou d'un trouble de l'apprentissage.

10. Composition pharmaceutique comprenant le dérivé peptidique agissant centralement selon l'une quelconque de la revendication 1 à la revendication 4 comme principe actif, dans laquelle la composition pharmaceutique est pour une administration intranasale.

11. Formulation nasale destinée à être utilisée selon la revendication 1, dans laquelle la partie qui agit sur un système nerveux central est choisie dans le groupe constitué par GLP-1, GLP-2 et la neuromédine U, la partie de séquence pénétrant la cellule est choisie parmi une oligoarginine représentée par Rn où n vaut de 6 à 12.
